# EUROPEAN PATENT APPLICATION

(11) **EP 2 322 123 A1**
(43) Date of publication of application: **18.05.2011**
(21) Application number: 09014235.7
(22) Date of filing: 13.11.2009
(51) Int. Cl.: A61F 9/007, A61B 3/10

(54) **Surgical device**

(71) Applicant: Carl Zeiss Surgical GmbH, 73447 Oberkochen (DE)
(72) Inventor: Hauger, Christoph, 73431 Aalen (DE)
(74) Representative: Diehl & Partner GbR

(57) **Abstract**

The method can be performed using a surgical instrument system (1) comprising: an OCT apparatus (3) including an interferometer; an optical fiber (7) coupled to the OCT apparatus and extending a probe arm of the interferometer; a hand tool (11) comprising a hand piece (9), a tube (13) extending away from the hand piece and having a distal end (21), wherein a distal portion of the optical fiber is received within the tube, a beam emitter coupled to the tip end of the optical fiber and configured to emit an OCT measuring beam into an emission direction; and an actuator (51) configured to change the emission direction of the OCT measuring beam relative to the tip end of the tube.

## Description

### Technical Field

The invention relates to surgical devices and surgical methods in general. In particular, the invention relates to a surgical instrument system and a surgical method for performing cataract surgery.

### Background Art

In a cataract surgery, a phakoemulsification needle is inserted into the capsular bag of a patient's eye through the cornea and the anterior chamber. Ultrasonic energy is supplied to the needle to emulsify the crystal lens of the eye. Emulsified pieces of the crystal lens are aspirated by the needle, and viscoelastic fluid is supplied through the needle into the capsular bag. An intraocular lens (IOL) is inserted into the capsular bag after the crystal lens has been removed by such processing.

In other eye surgery, a phakic intraocular lens (PIOL) is inserted into the anterior chamber of the eye or into the posterior chamber of the eye, without removing the crystal lens of the eye.

To obtain an optimal result in such surgery, where an intraocular lens is implanted into the anterior chamber, the posterior chamber or the capsular bag, it is important to select an intraocular lens of a size suitable for a patients eye. Lens sizes for anterior chamber phakic intraocular lenses and for posterior chamber phakic intraocular lenses have commonly been estimated by adding 0.5 to 1.0 mm to the horizontal white-to-white corneal diameter. However, the white-to-white corneal diameter is not a reliable indicator for the angle diameter of the interior chamber and the sulcus diameter, also referred to as sulcus-to-sulcus diameter, of the posterior chamber. In particular, it is difficult to determine the sulcus diameter and the diameter of the capsular bag from outside of the eye since the iris prevents optical measurements of the corresponding tissue portions of the eye.

It is desirable to provide methods and systems suitable for measuring geometries of eye tissues.

### Summary of the Invention

According to embodiments of the invention, a method comprises: inserting an OCT probe into an interior of an eye; emitting an OCT measuring beam from the OCT probe located within the eye into plural directions; and receiving OCT measuring light scattered from various tissue structures of the eye with the OCT probe.

The OCT probe is connected to an optical coherence tomography (OCT) apparatus for imaging body tissues. Since the OCT probe is located within the eye, high accuracy measurements of eye tissues can be performed. In particular, eye tissues located behind the iris can be imaged, and diameters of the sulcus and of the capsular bag can be precisely determined. Based on such determination, it is possible to select an intraocular lens from a stock of intraocular lenses having different sizes, and the selected intraocular lens can be inserted into the eye through a same incision in the cornea of the eye through which the OCT probe was inserted.

According to embodiments of the invention, a hole is formed in the crystal lens of the eye, and the OCT probe is inserted into this hole to measure the diameter of the capsular bag from within the crystal lens. Again, an intraocular lens can be selected from a stock of intraocular lenses having different sizes, and the selected intraocular lens can be inserted into the capsular bag after removal of the crystal lens.

According to embodiments of the invention, the optical probe is comprised by a surgical instrument system comprising: an OCT apparatus including an interferometer; an optical fiber coupled to the OCT apparatus and extending a probe arm of the interferometer; a hand tool comprising: hand piece, a tube extending away from the hand piece and having a distal end, wherein a distal portion of the optical fiber is received within the tube, a beam emitter coupled to the tip end of the optical fiber and configured to emit an OCT measuring beam into an emission direction; and an actuator configured to change the emission direction of the OCT measuring beam relative to the tip end of the tube.

The surgeon can manipulate the hand tool by grasping the hand piece with one or two hands to insert the tube into the eye such that the distal end of the tube is positioned at a location suitable for performing the OCT measurement of the eye tissues. The OCT measurement is performed by directing the OCT measuring beam into different directions from the emitter.

According to exemplary embodiments, the emitter is rotated about an axis by 360° to obtain an OCT B-Scan of one plane. The beam emitter is then translated by a small amount in a direction of extension of the distal portion of the tube, and a next B-Scan is obtained in a next adjacent plane. This process is repeated to obtain a sufficiently high number of B-Scans to generate an image of a sufficient volume of the eye tissues allowing to determine the desired geometric values of the eye, such as the diameter of the capsular bag, the diameter of the sulcus or the diameter of the anterior chamber angle.

According to exemplary embodiments, the hand tool also provides the function of a phakoemulsificator. For this purpose, the hand tool may comprise an ultrasonic transducer to vibrate a tip end of the tube or of a needle extending adjacent to the tube.

### Brief Description of the Drawings

The foregoing as well as other advantages features of the invention should become more apparent from the following detailed description of exemplary embodiments of the invention with reference to the accompanying drawings.
- Figure 1: is a schematic illustration of a surgical instrument system according to an embodiment of the invention;
- Figure 2: is a schematic illustration of a detail of the system shown in figure 1;
- Figure 3: is an illustration of a method of performing an OCT measurement of an anterior chamber of an eye;
- Figure 4: is an illustration of a method of performing an OCT measurement of a posterior chamber of an eye; and
- Figure 5: is an illustration of a method of performing an OCT measurement of a capsular bag of an eye

### Description of Exemplary Embodiments

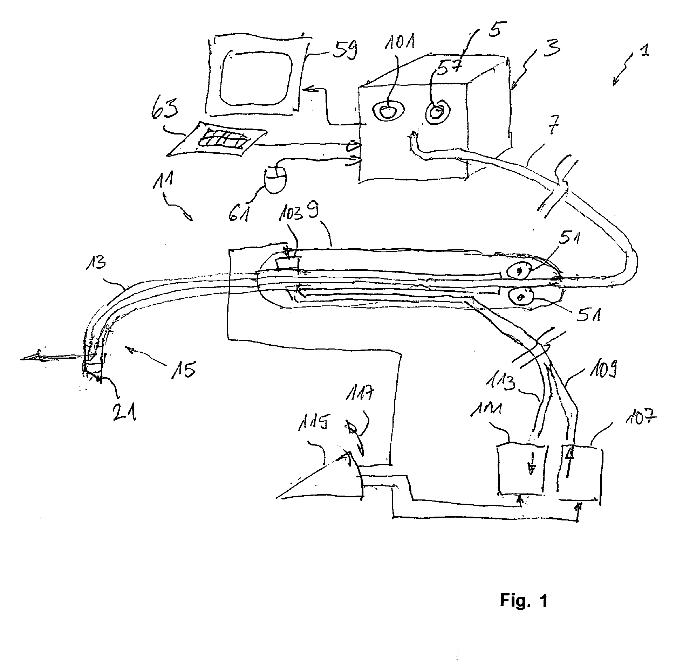

Figure 1 is a schematic illustration of a surgical instrument system according to an embodiment.

The surgical instrument system 1 comprises an optical coherence tomography (OCT) apparatus 3 including an optical interferometer received in a housing 5 and not shown in detail in figure 1. The interferometer includes a reference arm and a probe arm provided and extended by an optical fiber 7. The optical fiber comprises a fiber core for carrying the OCT measuring light. The fiber 7 extends through a hand piece 9 of a hand tool manipulated by a surgeon. The hand tool comprises a tube 13 extending away from the hand piece 9, wherein the optical fiber 7 is received in and extends through the tube 13 towards a distal end portion 15 of the tube.

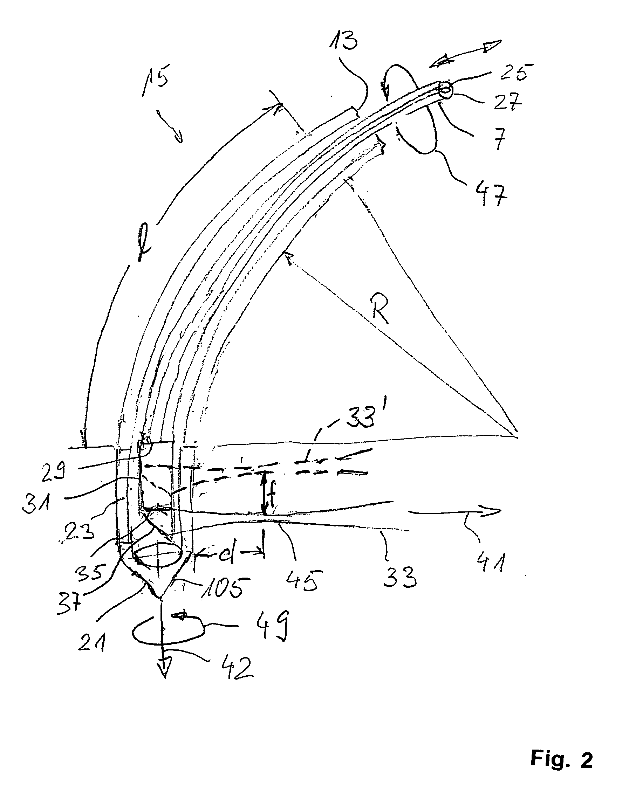

Figure 2 is a more detailed illustration of the distal end portion 15 of the tube 13. The distal end portion 15 of the tube 13 comprises a curved portion extending over a length 1 of more than 5 mm. Other exemplary values of the length 1 are 7 mm, 9 mm and 11 mm. A total length of the tube from the hand piece 9 to a tip end 21 thereof can be in a range from 10 mm to 50 mm. The curved portion may have a radius of curvature R within a range from 6 mm to 15 mm. Other exemplary values of the curvature range are 8 mm to 12 mm. The radius of curvature may be constant over the length 1, or the radius of curvature can vary over the length 1.

The tube 13 can be made of metal or of a plastic material providing a suitable rigidity to maintain its shape under usage. In the illustrated embodiment, a portion of the tube 13 close to its tip end 21 is made of a transparent material 23, such as glass or transparent plastic material.

A fiber core 25 and a fiber cladding 27 of the fiber 7 extend through the distal portion 15 of the tube 13 towards its tip end 21.

A tip end 29 of the fiber is coupled to a gradient index (GRIN) lens 31, which expands OCT measuring light supplied through the fiber 7 from the OCT apparatus 3 to shape a measuring beam 33 emitted from a front end 35 of the GRIN lens 31. A reflecting prism 37 is mounted to the front end 35 of the GRIN lens 31 and reflects the measuring beam such that is emitted in a direction 41 transverse to a direction of extension 42 of the distal end portion of the tube. An angle formed between the directions 41 and 42 can be 90° or greater, such as 100° or 120°, or smaller, such as 80° or 70°.

The GRIN lens 31 is configured such that a beam waist 45 of the OCT measuring beam 33 is formed at a distance d of about 6 mm away from the tube 13. Other exemplary values of the distance d are about 4 mm, about 7 mm and about 9 mm. It is also possible that the beam 33 emitted from the GRIN lens 31 is a collimated parallel beam.

The OCT apparatus comprises an actuator, such as a motor, to rotate the fiber 7 about its longitudinal axis as represented by an arrow 47 in figure 2. As a consequence, the GRIN lens 31 and prism 37 rotate about an axis oriented in direction 42 as indicated by an arrow 49 in figure 2. The actuator for rotating the fiber 7 can be located in the housing 5 or the hand piece 9. Background information with respect to OCT apparatuses including a rotating fiber can be obtained from US 6,134,003, the full disclosure of which is incorporated herein by reference.

Due to the rotation of the beam emitter formed by the GRIN lens and prism 37, the OCT measuring beam 33 also rotates about the tip end portion of the tube 13. While rotating the fiber 7 about 360°, the OCT apparatus 3 records one OCT B-Scan.

The hand tool 11 comprises an actuator, such as pair of rollers 51 driven by a motor controlled by the OCT apparatus 3 to displace the fiber 7 within the tube 13 in a longitudinal direction thereof. Figure 2 represents an OCT measuring beam 33' in broken lines which is emitted from the distal end portion of the tube 13 when the actuator 51 has retracted the fiber 7 by an amount f of e.g. 3 mm relative to the position indicated in full lines in figure 2.

By rotating the fiber 7 about its longitudinal axis and displacing the fiber 7 in its longitudinal direction, the OCT apparatus 3 can systematically scan a volume around the tip end 21 of the tube 13 and perform OCT imaging of that volume. Both the rotation and the displacement of the fiber can be carried out at constant velocities such that measuring data can be associated to with coordinates of tissue structures based on measuring times. It is, however, possible to allow for known non-constant rotational velocities and non-constant velocities of longitudinal movement if the data analysis is carried out with taking the velocities into account.

The scanning can be started by the surgeon by pressing, for example, a button 57 mounted on a front panel of the housing 5. A representation of the recorded OCT image of the OCT scan can be displayed, for example, on a monitor 59. Moreover, the surgeon can identify certain features in the image and perform measurements, such as distances measurements using, for example by using a mouse 61 or a keyboard 63, to move a cross-hair cursor or other pointer between portions of the image and features within the image and calculate distances between different features in the image.

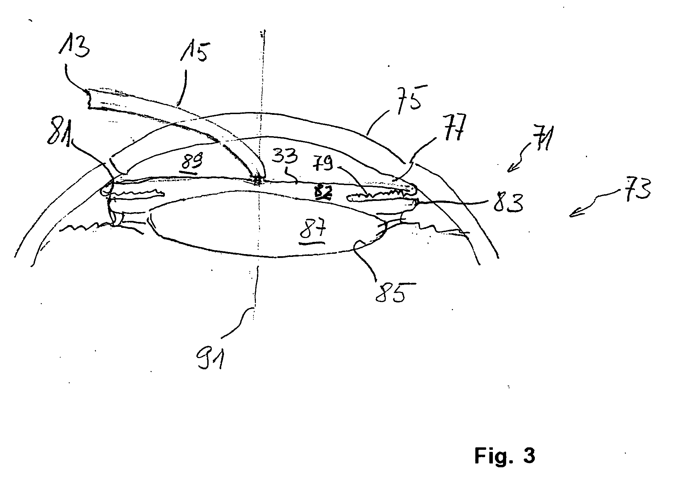

An embodiment of an OCT measuring method which can be performed using the system 1 is illustrated with reference to figure 3. Figure 3 shows an anterior portion 71 of a human eye 73 including a cornea 75, limbus 77, iris 79, anterior chamber 89, anterior chamber angle 81, posterior chamber 82, sulcus 83 and capsular bag 85 including a crystal lens 87. The distal end portion 15 of the tube 13 of the hand tool 11 is inserted through an incision in the cornea such that the tip end 21 of the tube 13 and beam emitter 31, 35 are positioned within the anterior chamber 89 of the eye 73. In particular, the distal end of the tube 13 is positioned close to a main axis 91 of the eye 73 and oriented such that the direction 42 is substantially parallel to the main axis 91. With such position of the hand tool 11, an OCT volume scan is performed as illustrated above. The volume scan includes various tissues of the eye 73 and in particular of the anterior chamber angle 81. A diameter of the anterior chamber angle 83 can be determined by the surgeon by interpreting and analyzing the recorded OCT images displayed on the monitor 59. Based on this determination of the angle diameter, the surgeon may select a suitable phakic intraocular lens from a stock of lenses having different diameters. The selected lens can be inserted into the anterior chamber through the same incision through with the tube 13 was previously inserted.

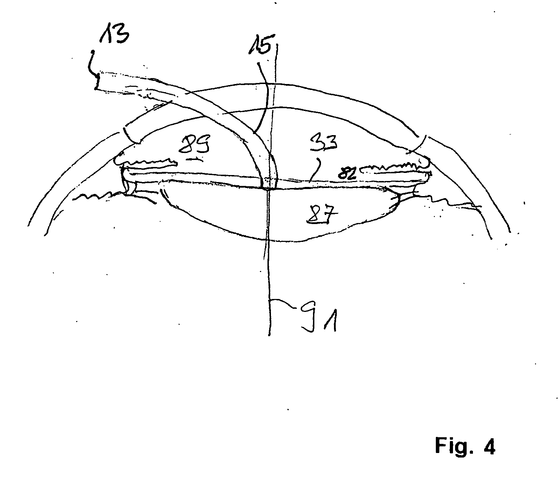

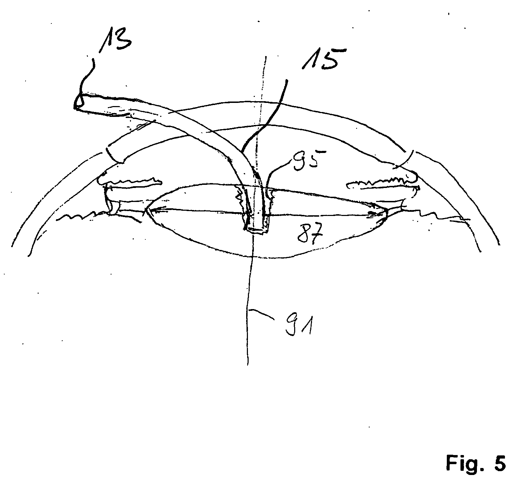

Figure 4 illustrates a further embodiment of the method. The method illustrated in figure 5 is similar to the method illustrated with reference to figure 3 above. However, the tip end 21 of the tube 13 is inserted into the posterior chamber 82 of the eye 73 such that it is positioned closer to the crystal lens 87 such that the beam 33 emitted from the distal end 21 of the tube 13 reaches the sulcus 81. For this purpose, it might be necessary to push the crystal lens a bit downward as illustrated in figure 4. By performing a volume scan with such positioning of the tip end of the tube 13 it is possible to image relevant portions of the sulcus 81 to be able to determine the sulcus diameter, also referred to as sulcus-to-sulcus diameter. Again, the surgeon may select a phakic intraocular lens of a suitable size from a stock of lenses having different sizes. The selected lens can be inserted into the posterior chamber through the same incision through which the tube 13 was inserted.

Figure 5 illustrates a further embodiment of an OCT measurement. In this embodiment, the tip end 21 of the tube 13 is positioned within the crystal lens 87 after an aperture or hole 95 has been formed in the lens 87 by some suitable method, such as phakoemulsification. The tube 13 is positioned such that the tip end substantially coincides with an axis 91 of the lens and is oriented substantially parallel thereto.

A OCT volume scan with such configuration of the tube 13 records an image of the capsular bag from which the surgeon can determine the diameter of the capsular bag 85 to select a suitable intraocular lens from a stock of lenses having different diameters.

The tube can be retracted from the eye 73, and the crystal lens 87 can be removed by phakoemulsification, for example. Thereafter, the surgeon can insert the selected intraocular lens into the capsular bag 85 through the same incision through which the tube 13 was previously inserted.

The actuator 51 can be controlled to withdraw the fiber 7 from within the tube 13 to a sufficient extend. For example, the tip end of the fiber can be positioned within the hand piece 9 after such withdrawal. This operation can be controlled by a button 101 mounted on the front panel of the housing 5 or some other input device, for example via the keyboard 63 or the mouse 61.

After such removal of the fiber from within the tube 13, the hand tool 11 can be operated as a phakoemulsificator. For this purpose, the hand tool comprises an ultrasonic transducer 103 coupled to the tube 13 such that the tip end of the tube starts vibrating at a high frequency. The tip end 21 of the tube carries a knife portion or sharp edge 105 suitable to cut emulsify body tissues, such as the crystal lens.

The surgical instrument system further comprises a supply 107 of viscoelastic fluid which is pumped into the tube 13 via a connecting tube 109. Further, the system 1 comprises a suction apparatus 111 connected to the tube 13 via a connecting tube 113. A foot paddle 115 which can be moved in a direction indicated by an arrow 117 in figure 1, or other input device can be used by the surgeon to operate the ultrasonic transducer 103, liquid supply 107 and suction apparatus 111 to perform phakoemulsification. For example, the hole 95 in the crystal lens 87 shown in figure 5 can be formed using the hand tool 11 which can be subsequently used to perform the OCT scan.

The present application discloses in particular the following features:
Features 1: A method comprising:
   inserting an OCT probe into an interior of an eye;
   emitting an OCT measuring beam from the OCT probe located within the eye into plural directions; and
   receiving OCT measuring light scattered from various tissue structures of the eye with the OCT probe.
Features 2: The method according to features 1, further comprising inserting an intraocular lens into the eye.
Features 3: The method according to features 1 or 2, further comprising determining at least one of a diameter of an anterior chamber of the eye, a diameter of a posterior chamber of the eye and a diameter of a capsular bag of the eye based on the received OCT measuring light.
Features 4: The method according to features 1, further comprising selecting an intraocular lens from a stock of intraocular lenses having different diameters based on the determined diameter, and inserting the selected intraocular lens into the eye.
Features 5: The method according to one of features 2 to 4, wherein the inserting comprises inserting the intraocular lens into one of the anterior chamber of the eye, the posterior chamber of the eye and the capsular bag of the eye.
Features 6: The method according to one of features 2 to 5, further comprising retracting the OCT probe before inserting the intraocular lens.
Features 7: The method according to one of features 2 to 6, further comprising generating an opening in a cornea of the eye and inserting the OCT probe through the opening in the cornea into the interior of the eye.
Features 8: The method according to features 7, further comprising inserting an intraocular lens into the eye through the opening.
Features 9: A method, in particular in features with one of features 1 to 8, the method comprising:
   forming a hole in a lens of an eye by removing a portion of the lens;
   inserting an OCT probe into the hole formed in the lens; emitting an OCT measuring beam from the OCT probe located within hole formed in the lens into plural directions; and receiving OCT measuring light scattered from various tissue structures of the eye with the OCT probe.
Features 10: The method according to features 9, wherein the forming of the hole in the lens comprises phakoemulsification.
Features 11: The method according to one of features 9 or 10, further comprising removing of the lens.
Features 12: The method according to features 11, wherein the removing of the lens comprises phakoemulsification.
Features 13: The method according to features 12, further comprising inserting an intraocular lens into the capsular bag of the eye.
Features 14: The method according to one of features 9 to 13, further comprising determining a diameter of a capsular bag of the eye based on the received OCT measuring light.
Features 15: The method according to features 14, further comprising selecting an intraocular lens from a stock of intraocular lenses having different diameters based on the determined diameter, and inserting the selected intraocular lens into the eye.
Features 16: The method according to one of features 1 to 15, wherein the OCT probe comprises a tube having a proximal end and a distal end, and an optical fiber extending through the tube, wherein the OCT measuring beam is emitted from the distal end of the tube.
Features 17. The method according to features 16, further comprising moving the optical fiber to direct the OCT measuring light into the different directions.
Features 18: The method according to features 17, wherein the moving of the optical fiber comprises rotating the fiber about a longitudinal axis thereof.
Features 19: The method according to features 17, wherein the moving of the optical fiber comprises bending a distal end of the optical fiber.
Features 20: The method according to features 19, wherein the bending of the distal end of the optical fiber comprises oscillating a distal end portion of the optical fiber.
Features 21: The method according to one of features 16 to 20, wherein the OCT measuring beam is emitted in a direction transverse to a direction of extension of a distal portion of the optical fiber.
Features 22: The method according to one of features 16 to 21, wherein a GRIN lens is attached to the distal end of the fiber.
Features 23: The method according to features 22, wherein the GRIN lens carries a reflecting surface directing the OCT measuring beam into a direction transverse to a direction of extension of the distal portion of the optical fiber.
Features 24: The method according to one of features 16 to 23, wherein the inserting of the OCT probe into the interior of the eye comprises orienting the distal end of the tube such that it is oriented substantially parallel to a main axis of the eye.
Features 25: The method according to one of features 16 to 24, wherein the inserting of the OCT probe into the interior of the eye comprises positioning the distal end of the tube such that it is located substantially on a main axis of the eye.
Features 26: The method according to one of features 16 to 25, wherein the distal end portion of the tube is a curved portion.
Features 27: The method according to features 26, wherein the curved portion has a same curvature over a length greater than at least one of 5 mm, 7 mm, 9 mm and 11 mm.
Features 28: The method according to features 26 or 27, wherein the curved portion has a radius of curvature in a range from 6 mm to 15 mm, and in particular in a range from 8 mm to 12 mm.
Features 29: The method according to one of features 16 to 28, wherein the tube is integrated with a phacoemulsificator.
Features 30: The method according to features 29, further comprising retracting the optical fiber from within the tube before performing phakoemulsification.

While certain exemplary embodiments are disclosed herein, alternatives, modifications and variations will be apparent to those skilled in the art. Accordingly, the exemplary embodiments set forth herein are intended to be illustrative and not limiting in any way. Various changes may be made without departing from the spirit and scope of the present disclosure.

## Claims

1. A surgical instrument system comprising:
an OCT apparatus including an interferometer;
an optical fiber coupled to the OCT apparatus and
extending a probe arm of the interferometer;
a hand tool comprising:
hand piece,
a tube extending away from the hand piece and having a distal end, wherein a distal portion of the optical fiber is received within the tube,
a beam emitter coupled to the tip end of the optical fiber and configured to emit an OCT measuring beam into an emission direction; and
an actuator configured to change the emission direction of the OCT measuring beam relative to the tip end of the tube.

2. The surgical instrument system according to claim 1, wherein the actuator is configured to rotate the optical fiber about a longitudinal axis thereof.

3. The surgical instrument system according to claim 1, wherein the actuator is configured to move the optical fiber in a longitudinal axis thereof relative to the tube.

4. The surgical instrument system according to claim 1, wherein the actuator is configured to rotate the optical fiber about a longitudinal axis thereof and to move the optical fiber in the longitudinal axis relative to the tube.

5. The surgical instrument system according to one of claims 1 to 4, wherein the distal end portion of the tube is a curved portion.

6. The surgical instrument system according to claim 5, wherein the curved portion has a same curvature over a length greater than at least one of 5 mm, 7 mm, 9 mm and 11 mm.

7. The surgical instrument system according to claim 5 or 6, wherein the curved portion has a radius of curvature in a range from 6 mm to 15 mm, and in particular in a range from 8 mm to 12 mm.

8. The surgical instrument system according to one of claims 1 to 4, wherein the beam emitter comprises a GRIN lens.

9. The surgical instrument system according to one of claims 1 to 8, wherein the beam emitter comprises a reflector.

10. The surgical instrument system according to one of claims 1 to 9, wherein the beam emitter is configured to shape the OCT measuring beam such that it has a beam waist at a distance of greater than 4 mm and less than 9 mm from the emitter.

11. The surgical instrument system according to one of claims 1 to 10, wherein the optical fiber is retractable from within the tube.

12. The surgical instrument system according to one of claims 1 to 11, wherein the hand tool comprises a phacoemulsificator.

13. The surgical instrument system according to one of claim 12, wherein phacoemulsificator comprises an ultrasonic transducer configured to vibrate the tip end of the tube.

14. The surgical instrument system according to claim 13, wherein the tip end of the tube includes a needle.

15. The surgical instrument system according to one of claims 12 to 14, wherein the phacoemulsificator comprises a fluid supply and/or an aspiration line.
